(19)

Europäisches
Patentamt
European
Patent Office
Office européen
des brevets

(11) **EP 3 287 145 A1**

(12) **EUROPEAN PATENT APPLICATION**

(43) Date of publication:
**28.02.2018 Bulletin 2018/09**

(21) Application number: **16185880.8**

(22) Date of filing: **26.08.2016**

(51) Int Cl.:
*A61K 47/36* [(2006.01)]  *A61K 9/14* [(2006.01)]
*A61K 35/745* [(2015.01)]  *A61K 35/747* [(2015.01)]
*A23L 29/30* [(2016.01)]  *A23L 33/135* [(2016.01)]
*C08L 3/02* [(2006.01)]  *C08B 30/18* [(2006.01)]

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **DuPont Nutrition Biosciences ApS
1411 Copenhagen K (DK)**

(72) Inventor: **HOLLARD, Christophe
Mc Farland, WI 53558 (US)**

(74) Representative: **DuPont EMEA
Langebrogade 1
1411 Copenhagen K (DK)**

(54) **USE OF MALTODEXTRIN AS AN EXCIPIENT**

(57)     The present invention relates to the use of maltodextrin as an excipient. In particular, the present invention relates to the use of maltodextrin as an excipient in probiotic formulations.

The present invention also relates to a method to provide a probiotics formulation with improved dispersibility and/or shelf life comprising adding maltodextrin to probiotic formulations.

**EP 3 287 145 A1**

**Description**

**FIELD OF THE INVENTION**

The present invention relates to a use. In particular the present invention relates to the use of maltodextrin as an excipient. Further, the present invention relates to a method to provide a probiotic formulation with improved dispersibility and/or shelf life comprising adding maltodextrin to probiotic formulations

**BACKGROUND OF THE INVENTION**

Probiotics can be consumed by dissolving a powder containing the probiotics into a liquid, like water in a glass, or by directly pouring the powder into a person's mouth. In both applications, the powder must dissolve rapidly into the liquid or into the mouth's saliva to provide an acceptable commercial product and provide the proper healthy dose. In addition, the final consumers usually prefer to have a transparent solution when the probiotic powder is dispersed and dissolved into a liquid. Furthermore, the healthy dose in the finish format must be guaranteed from the beginning to the end of shelf-life.

Probiotics in finish format are sold to the final consumers with specific doses of probiotics that provide given health effects. Excipients are used as bulking agents when making capsules, sachets or other finished formats with the appropriate healthy dose.

The choice of the excipients is then critical to provide a probiotic powder with an optimal transparency, dispersibility, and shelf-life stability.

A number of attempts have been made to find excipients that offer an optimal level of transparency, dispersibility, particle size and shelf-life stability when mixed with probiotics

Known excipients include microcrystalline cellulose (MCC), amylum, magnesium sulphate, calcium lactate, sodium citrate, polyvinyl pyrrolidone and sucrose.

However, these excipients do not fulfill the requirement of an optimal excipient due to the fact that they are not soluble or difficult to dry.

Hence it is an object of the invention to provide an excipient which overcomes the above mentioned disadvantages.

The present invention alleviates the problems of the known excipients and provides a new use of a polysaccharide as an excipient. It has thus unexpectedly been found that a polysaccharide with a specific depolymerization level and a specific range of humidity significantly improves transparency, dispersibility and shelf life stability of probiotic powders.

**SUMMARY OF THE INVENTION**

In a first aspect, the present invention relates to the use of maltodextrin as an excipient, wherein said maltodextrin has a dextrose equivalent (DE) between 17 and 23 and a water activity (Aw) below 0.1.

In a second aspect, the invention relates to a method to provide a probiotics formulation with improved dispersibility and/or shelf life comprising adding maltodextrin to probiotics formulations, wherein said maltodextrin has a dextrose equivalent (DE) between 17 and 23 and a water activity (Aw) below 0.1.

In a third aspect, the invention relates to a method for producing a maltodextrin having a dextrose equivalent (DE) between 17 and 23 and a water activity (Aw) below 0.1 comprising the step of drying These and other aspects of the present invention will now be explained in more detail. In this regard, the particular features mentioned with respect to each aspect are not necessarily limited to that aspect and may be combined with other features of the same or other aspects as will be appreciated by one skilled in the art.

DETAILED DISCLOSURE OF THE INVENTION

In one aspect the present invention relates to the use of maltodextrin as an excipient, wherein said maltodextrin has a dextrose equivalent (DE) between 17 and 23 and a water activity (AW) below 0.1.

An excipient, as herein defined, refers to a substance formulated alongside the active ingredient and it is included for the purpose of long-term stabilization, bulking up solid formulations that contain potent active ingredients or to confer a therapeutic enhancement on the active ingredient in the final dosage form, such as by enhancing solubility. The excipient can be referred to as "bulking agents", "fillers", or "diluents". It can be used when making capsules, sachets or other formats with an appropriate healthy dose of the active ingredient.

Dextrose equivalent (DE) as herein defined refers to a measure of the amount of reducing sugars present in a sugar product, relative to dextrose (glucose), expressed as a percentage on a dry basis. For example, a maltodextrin with a DE of 10 would have 10% of the reducing power of dextrose (which has a DE of 100). Thus The DE describes the degree of conversion of starch to dextrose, and starch is having a DE close to zero, glucose/dextrose is 100 (percent), dextrins vary between 1 and 13, while maltodextrins vary between 3 and 20 and glucose syrups contain a minimum of 20% reducing sugars.

Maltodextrin as herein defined consists of D-glucose units connected in chains of variable length. The glucose units are primarily linked with $\alpha(1\rightarrow4)$ glycosidic bonds. Maltodextrin is typically composed of a mixture of chains that vary from 3 to 17 glucose units long.

In one embodiment the maltodextrin has a dextrose equivalent (DE) between 17 and 23. In one embodiment the maltodextrin has a dextrose equivalent (DE) between 15 and 20. In one embodiment the maltodextrin has a dextrose equivalent (DE) between 16 and 20. In one embodiment the maltodextrin has a dextrose equivalent (DE) between 17 and 20. In one embodiment the maltodextrin has a dextrose equivalent (DE) between 17 and 21. In one embodiment the maltodextrin has a dextrose equivalent (DE) between 17 and 22. In one embodiment the maltodextrin has a dextrose equivalent (DE) between 18 and 20. In one embodiment the maltodextrin has a dextrose equivalent (DE) between 19 and 20. In one embodiment the maltodextrin has a dextrose equivalent (DE) of 20.

The water activity Aw is the partial vapor pressure of water in a substance divided by the standard state partial vapor pressure of water. In one embodiment the water activity (Aw) is below 0.1. In one embodiment the water activity (Aw) is below 0.09.

In one embodiment the maltodextrin is used in probiotics formulations. "Probiotics" or "probiotic strain(s)" refers to strains of bacteria, which have a beneficial effect on the host when ingested by a subject and which are generally regarded as safe (GRAS) to humans.

The term 'probiotic' is thus defined as covering any non-pathogenic microorganism which, when administered live (e.g. viable) in adequate amounts, confer a health benefit on the host. These probiotic strains generally have the ability to survive the passage through the upper part of the digestive tract. They are non-pathogenic, non-toxic and exercise their beneficial effect on health on the one hand via ecological interactions with the resident flora in the digestive tract, and on the other hand via their ability to influence the immune system in a positive manner via the "GALT" (gut-associated lymphoid tissue).

Depending on the definition of probiotics, these microorganisms, when given in a sufficient number, have the ability to progress live through the intestine, however they do not cross the intestinal barrier and their primary effects are therefore induced in the lumen and/or the wall of the gastrointestinal tract. They then form part of the resident flora during the administration period. This colonization (or transient colonization) allows the probiotic microorganism to exercise a beneficial effect, such as the repression of potentially pathogenic micro-organisms present in the flora and interactions with the immune system of the intestine.

In one embodiment the probiotic is a bacterium of the species *Lactobacillus acidophilus* or a mixture thereof.

In another embodiment the probiotic is a bacterium of the species *Bifidobacterium lactis* or a mixture thereof.

Lactobacillus acidophilus and Bifidobacterium ssp. are the most commonly used probiotics. Their presence in the gastrointestinal tract aids in maintaining a desirable gastrointestinal microbiota, helps in preventing infection by pathogenic bacteria and is in other ways of benefit to the host.

The bacteria of the species *Lactobacillus acidophilus and of* the species *Bifidobacterium lactis are well known by the skilled person in the art and commercially available.*

In one embodiment the formulations are provided in a powder form.

In another embodiment the maltodextrin improves the shelf life of the probiotics formulation.

In one embodiment the maltodextrin is combined with a base.

The combination of a base with the maltodextrin is used to adjust the pH. The pH has a direct effect on the stability of the probiotic composition. In one embodiment the pH is between 4-9. In one embodiment the pH is between 5-8 and in another embodiment the pH is between 6-7.

In another embodiment when the maltodextrin is combined with a base, the base is selected from Sodium hydroxide (NaOH), Potassium hydroxide (KOH) or Ammonium hydroxide ($NH_4OH$).

In one embodiment the maltodextrin improves the dispersibility of the probiotics formulation.

In one embodiment the maltodextrin is a potato maltodextrin.

In a second aspect there is provided a method to provide a probiotics formulation with improved dispersibility and/or shelf life comprising adding maltodextrin to probiotic formulations, wherein said maltodextrin has a dextrose equivalent (DE) between 17 and 23 and a water activity (Aw) below 0.1.

In one embodiment of the method to provide a probiotics formulation with improved dispersibility and/or shelf life the maltodextrin is added as an excipient.

In one embodiment of the method to provide a probiotics formulation with improved dispersibility and/or shelf life, the probiotic is a bacterium of the species *Lactobacillus acidophilus* or a mixture thereof.

In a further embodiment of the method to provide a probiotics formulation with improved dispersibility and/or shelf life, the probiotic is a bacterium of the species *Bifidobacterium lactis* or a mixture thereof.

In one embodiment of the method to provide a probiotics formulation with improved dispersibility and/or shelf life, the probiotics formulations are in a powder form.

In one embodiment of the method to provide a probiotics formulation with improved dispersibility and/or shelf life, the maltodextrin is a potato maltodextrin.

In a third aspect there is provided a method for producing a maltodextrin having a dextrose equivalent (DE) between 17 and 23 and a water activity (Aw) below 0.1 comprising the step of drying.

In a further embodiment of the method for producing a maltodextrin the drying is performed in a fluidized bed or by spray drying.

**FIGURES**

The present invention will now be described with reference to the following non-limiting examples.

**BRIEF DESCRIPTION OF THE FIGURES**

**Figure 1:** Dissolution profile of different polysaccharides.

**Figure 2:** Aw impact on the Lactobacillus acidophilus (NCFM) stability.

**Figure 3:** Aw impact on the *Bifidobacterium* lactis (strain BBL) stability.

**Figure 4:** Aw impact on the *Bifidobacterium* lactis (Strain BBI) stability.

**Figure 5:** Cell recovery at 30 C after 30 days for various maltodextrin pH for various strains of probiotics.

**EXAMPLES**

**Example 1. Transparency and dissolution rate of polysaccharide excipients**

Various polysaccharide excipients were obtained for transparency and dissolution rate evaluation.

**1.1 Materials**

The products that were tested are shown in Table 1 which include potato maltodextrin at various levels of depolymerization (or dextrose equivalent (DE)), some rice maltodextrin at various levels of depolymerization, microcrystalline cellulose, and potato starch.

**Table 1: Evaluated polysaccharide excipients**

| Polysaccharides tested | Suppliers | Characteristics |
|---|---|---|
| MD 2 | AVEBE | Potato maltodextrin DE 2 |
| MD 6 | AVEBE | Potato maltodextrin DE 6 |
| MD 10 | AVEBE | Potato maltodextrin DE 10 |
| MD 20 | AVEBE | Potato maltodextrin DE 20 |
| MCC | AVICEL | microcrystalline cellulose |
| Instarice | Primera Foods | Rice agglomerated maltodextrin (DE 16?) |
| Benefiber | Benefiber | Wheat dextrin |
| Prejel PA 5 PH | DFE Pharma | Fully pregelatinised potato starch |
| Maltrin Or R170 | Grain processing corporation | Rice maltodextrin (DE=12) |
| Rice trim 35 | Primera Foods | Rice maltodextrin (DE=35) |
| Solani amylum | AVEBE | Potato starch |

**1.2 Methods**

The transparency was evaluated by measuring the optical density of solution in which the excipients was fully dissolved and the dissolution rate was tested by measuring the optical density (OD) as a function of time. The method described below simulates what happens when excipients are dissolved into a glass of water.

A small beaker wrapped with an aluminum foil was filled with 200 ml of tap water at 25°C and stirred at 600 rpm with a cross-shaped bar activated by a magnetic stirrer. The turbidimeter ASD19-N from Optek inline control (Optek-converter FC20 with storage time interval 1s) was installed on the side of the container. 3.5 gr of sample was added on the surface of the liquid.

The transparency of the dissolved excipients was evaluated by measuring the optical density after 600 sec (or 10 minutes) of stirring.

The dissolution rate was followed by measuring the OD as a function of time. The results were expressed as a percent of OD divided by the maximum OD obtained during the run and also expressed as OD for various mixing times.

**1.3 Transparency results of dissolved excipients.**

Table 2 shows the optical density of the various excipients after 600 sec of dissolution in water.

**Table 2: OD after 600 sec of dissolution for various excipients**

| Polysaccharides tested | OD after 600 s |
|---|---|
| MD2 | 0.011 |
| MD6 | 0.022 |
| MD10 | 0.037 |
| MD20 | 0.000 |
| MCC | 1.300 |
| Instarice MR211 | 0.135 |
| Benefiber | 0.006 |
| Maltrin OR R170 | 0.000 |
| Rice trim 35 | 0.057 |
| Solani amylum | 0.794 |
| Prejel PA 5 PH | 0.004 |

As shown in Table 2, the four most transparent dissolved excipients are MD 20, Benefiber, Maltrin R170, and Prejel. The dissolution rates of those excipients were then measured.

**1.4 Dissolution rates results.**

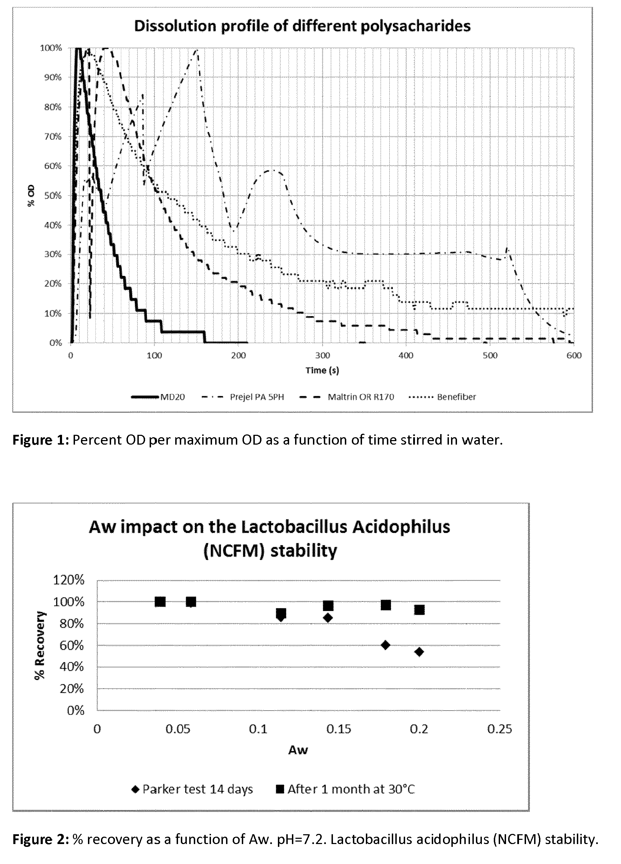

Figure 1 shows the percent of OD measured every second divided by the maximum OD as a function of time for the four excipients with the best transparencies in water.

In a first step, the percent OD increases very rapidly corresponding to a phase during which the powder is dispersing in the water column, then in a second phase the OD percent drops as the excipient is being dissolved. The percent OD drops very quickly for fast dissolving excipients like MD 20, other like Prejel PA 5PH the powder creates some lumps during the dispersion phase; the lumps then absorb strongly when passing in front of the OD reader which explains the successive peaks of absorption.

The OD measurement at various stirring time are then reported in Table 3 for all the excipients

**Table 3: OD in the water column at various stirring time**

| Polysaccharides tested | Time (s) | | | |
|---|---|---|---|---|
| | 60 | 120 | 160 | 180 |
| MD 2 | 0.080 | 0.063 | 0.053 | 0.047 |

(continued)

| Polysaccharides tested | Time (s) | | | |
|---|---|---|---|---|
| | 60 | 120 | 160 | 180 |
| MD 6 | 0.164 | 0.175 | 0.161 | 0.153 |
| MD 10 | 0.112 | 0.136 | 0.117 | 0.117 |
| MD 20 | 0.006 | 0.001 | 0.000 | 0.000 |
| MCC | 1.044 | 1.215 | 1.240 | 1.246 |
| Instarice | 0.128 | 0.134 | 0.135 | 0.135 |
| Benefiber | 0.033 | 0.022 | 0.018 | 0.016 |
| Prejel PA 5PH | 0.653 | 0.791 | 0.399 | 0.054 |
| Maltrin OR R170 | 0.060 | 0.027 | 0.020 | 0.017 |
| Rice trim 35 | 0.590 | 0.038 | 0.034 | 0.034 |
| Solani amylum | 0.642 | 0.728 | 0.739 | 0.744 |

Table 3 clearly shows that the potato maltodextrin with a dextrose equivalent of 20 (MD20) has the fastest dissolution rate and the highest transparency.

**Example 2: Transformation of potato maltodextrin with a DE of 20 to improve probiotic shelf-life stability.**

Potato maltodextrin with a DE 20 is clearly the best polysaccharide excipient for dissolution in water or in the mouth. However, the Activity of water (Aw) and acidity measured as pH of MD 20 may not be at its optimum for shelf-life stability. Some additional transformation may need to be done to improve those **parameters** and **improve** probiotic shelf-life when mixed with MD 20.

**2.1 Method**

**2.1.1 Aw measurement**

An Aqualab 4TE, Decagon was used for the measurements of water activity. The sample (about 1g) was equilibrated within the headspace of a sealed chamber containing a mirror, an optical sensor, an internal fan and an infrared temperature sensor.

**2.1.2 pH measurement:**

A C6010 Consor was used for the measurement of pH. A solution was prepared at 100 g/l using demineralized water at 25°C.

**2.1.3 Method to change the pH of maltodextrin:**

**FB Equipment description**

All trials were carried out on a GLATT Procell fluid bed dryer. The apparatus was equipped with the GF3 insert and the nozzle (1.2 mm of diameter) was placed in a top position. The inlet air used was taken from a clean room air with controlled humidity (40%RH) and was dehumidified at 1g H2O / Kg air with a dehumidifier Munters L180E.

**Process parameters and formulations used**

The process parameters and formulations are described in table 4.

**Table 4: Process parameters and formulations used**

| Sample number | % solutionr added | type of solution | Mass MD20 loaded | Spray rate (g/min) | Nozzle pressure (bar) | Airflow (m3/h) | Inlet temperature (°C) | Product temperature (°C) | Yield | Post drying time (min) |
|---|---|---|---|---|---|---|---|---|---|---|
| 15186B | 25% | Demineralized water | 2000 | 13.8 | 2.5 | 100 | 68 | 45.5 | 5% | 24 |
| 15186E | 22% | 1% NaOH 1N | 2000 | 13.5 | 2.5 | 100 | 68 | 43 | 8% | 8 |
| 15202A | 26% | 25% NaOH 1N | 2000 | 13.5 | 2.5 | 100 | 68 | 46 | 19% | 22 |
| 15203A | 28% | 1.86 % NaOH 1N | 2000 | 15.4 | 2.5 | 100 | 68 | 43 | 22% | 24 |
| 15204A | 25% | 3.66 % NaOH 1N | 2000 | 14.7 | 2.5 | 100 | 68 | 46 | 10% | 24 |
| 15204B | 28% | 3.1% NaOH 1N | 2000 | 14.8 | 2.5 | 100 | 68 | 46.6 | 6% | 23 |

The final MD 20 pH's obtained are shown in table 5 below.

**Table 5: MD 20 pH obtained**

| Sample number | Water activity (Aw) | pH |
|---|---|---|
| 15186B | 0.078 | 4.95 |
| 15186E | 0.088 | 5.84 |
| 15202A | 0.087 | 10.14 |
| 15203A | 0.09 | 6.81 |
| 15204A | 0.073 | 7.44 |
| 15204B | 0.076 | 7.23 |

The data demonstrates that by the use of the described process it is possible to make potato maltodextrin with a pH varying from 4.95-10.14

### 2.1.4 Method to change Aw

Samples with various water activity levels were prepared in the fluidized bed reactor by adjusting the inlet temperature and drying time, as shown in Table 6. pH is held constant at pH 7.2.

**Table 6: Water activity MD 20 samples obtained**

| Sample number | Water activity (Aw) |
|---|---|
| 1 | <0.025 |
| 2 | 0.034 |
| 3 | 0.093 |
| 4 | 0.138 |
| 5 | 0.181 |
| 6 | 0.222 |

It is thus demonstrated that it is possible to prepare a potato maltodextrin with an Aw varying from 0.025-0.222 by the use of the described process.

### 2.1.5 Accelerated shelf-life method

The experiments were conducted with the following freeze dried probiotics: Lactobacillus acidophilus (NCFM strain), *Bifidobacterium lactis* (strain BBI), *Bifidobacterium lactis* (strain BBL). The various probiotic species were blended with MD 20 samples equilibrated at various pH and Aw to make a blend with 30 percent of probiotics. The blends were mixed by rotation (about 60tr/min) in plastic bottle during 20 min. Then, the sealed foil bags were filled with the different blends. The preparation of the samples was made in a clean room at 40% RH and 25 deg. C.

The sealed foil bags with the blends were stored in an environmental chamber at 30°C for 3 months or in an environmental chamber at 30°C, 65% HR; or in a chamber at 38 C for 2 weeks (known as Parker test). CFU and Aw were measured at time 0, 1, 3 months to evaluate the impact of excipient Aw and pH on stability performance.

### Cell count method:

1 g of sample was precisely weighed in a bottle; then sterile peptone water was added into the bottle up to 100g and was mixed for 5 minutes at 400 rpm using a bench top shaker, then the samples was let to rehydrate for 20 minutes at room temperature and mixed afterward for 5 minutes to obtain a homogenous solution. A $10^{-2}$ dilution from the original sample was obtained.

Subsequent dilutions were carried out at 1:10 steps and were made by adding 1 ml of the solution to 9 ml of peptone water. The solutions were homogenized at each step during 20 seconds using a Vortex system at maximum speed.

MRS agar with 1% of cysteine was used to plate cells.

For accuracy of determination, it is well known in the art that there must be between 30 and 300 colonies on each agar plate. It is therefore important to estimate the cell concentration of the sample to be analyzed in order to achieve countable plates without having to do too many dilutions.

For each determination, 4 plates were counted: two different volumes of cell suspension were plated and each volume was made in duplicate. Then the number of colonies obtained on the plates were added and divided by the total sum of the volumes of dilution used for these plates.

The plates were then incubated at 37°C for 72 hours and colonies counted.

**Calculation method to determinate the recovery**

Probiotic recovery rate was expressed in two different ways.

a) Percent recovery

$$\% \text{ Recovery} = (\text{Colony Forming Unit (CFU) after storage} / \text{CFU t0})*100$$

b) Log loss

$$\text{Log loss} = \text{Log (CFU t0)} - \text{Log (CFU after storage}$$

**2.2 Experimental results**

**2.2.1 Stability as a function of Aw**

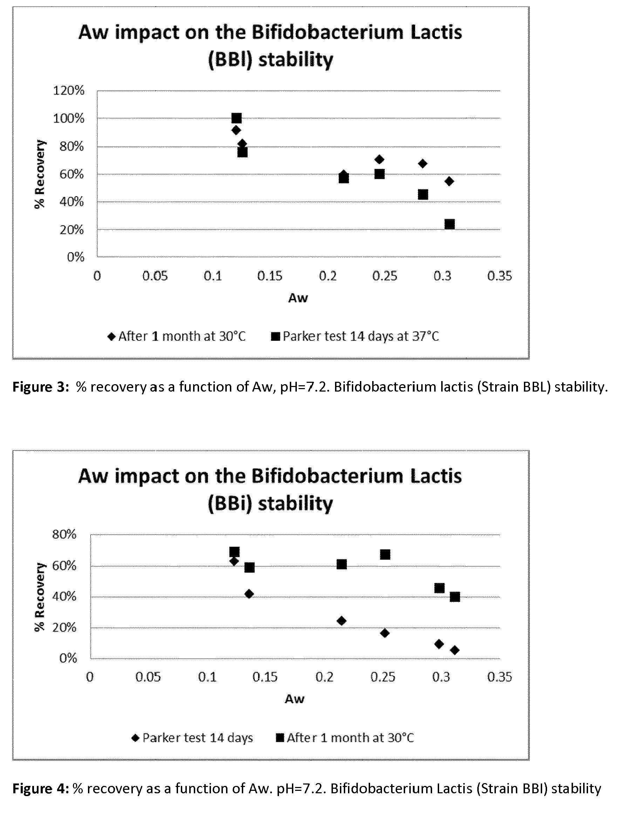

Figures 2 to 4 shows the stability of the probiotic cultures Lactobacillus acidophilus (NCFM strain), *Bifidobacterium lactis* (strain BBL), *Bifidobacterium lactis* (strain BBI) as a function of Aw, measured at pH7.2.

It is clearly demonstrated that when the water activity is higher than 0.1 the recovery percent decreases. However, as an example when the water activity is below 0.1 a 100% recovery is obtained for the Lactobacillus acidophilus strain even after 1 month at 30C, see figure 2.

The figures demonstrate that the recovery percentage decreases as the Aw increases.

**2-2-2 Stability as a function of pH**

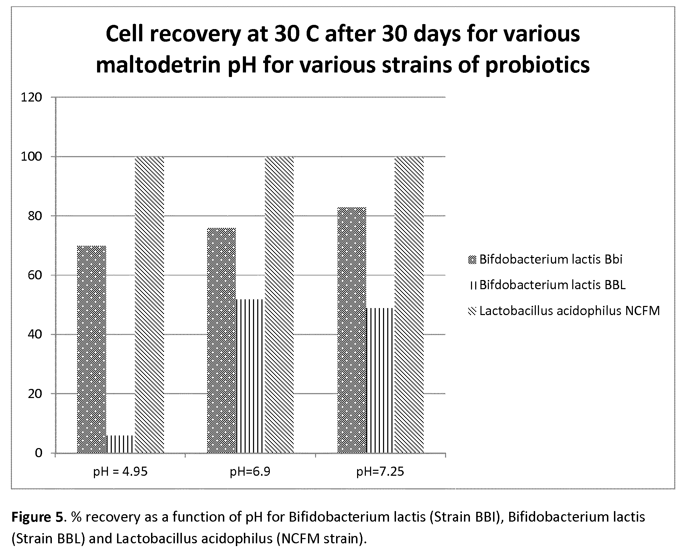

Figure 5 shows the recovery percent as a function of pH 4.95, 6.90 and 7.25 for *Bifidobacterium lactis* (strain BBI), *Bifidobacterium lactis* (strain BBL) and *Lactobacillus acidophilus* (NCFM strain). It can be seen from figure 5 that certain strains like BBI and BBL are sensitive to the pH of the excipients, and that BBL is having an optimal pH at 6.9 and BBI at 7.25.

**Claims**

1. Use of maltodextrin as an excipient, wherein said maltodextrin has a dextrose equivalent (DE) between 17 and 23 and a water activity (Aw) below 0.1.

2. The use according to claim 1, wherein the maltodextrin is used in probiotics formulations.

3. The use according to claim 2, wherein the probiotic is a bacterium of the species *Lactobacillus acidophilus* or a mixture thereof.

4. The use according to claim 2, wherein the probiotic is a bacterium of the species *Bifidobacterium lactis* or a mixture thereof.

5. The use according to any one of the claims 1 to 4, wherein the formulations are in the powder form.

6. The use according to any one of the claims 1 to 5 wherein the maltodextrin improves the shelf life of the probiotics formulation.

7. The use according to any one of the claims 1 to 6 wherein the maltodextrin is combined with a base.

8. The use according to claim 7, wherein the base is selected from Sodium hydroxide (NaOH), Potassium hydroxide (KOH) or Ammonium hydroxide (NH$_4$OH)

9. The use according to any one of the claims 1 to 8 wherein the maltodextrin improves the dispersibility of the probiotics formulation.

10. The use according to any one of the claims 1 to 9, wherein the maltodextrin is potato maltodextrin.

11. Method to provide a probiotics formulation with improved dispersibility and/or shelf life comprising adding maltodextrin to probiotic formulations, wherein said maltodextrin has a dextrose equivalent (DE) between 17 and 23 and a water activity (AW) below 0.1.

12. The method according to claim 11, wherein the maltodextrin is added as an excipient.

13. The method according to any of claims 11 to 12, wherein the probiotic is a bacterium of the species *Lactobacillus acidophilus* or a mixture thereof.

14. The method according to any of claims 11 to 12, wherein the probiotic is a bacterium of the species *Bifidobacterium lactis* or a mixture thereof.

15. The method according to any of claims 11 to 14, wherein the probiotics formulations are in the powder form.

16. The method according to any one of the claims 11 to 15, wherein the maltodextrin is potato maltodextrin.

17. A method for producing a maltodextrin having a dextrose equivalent (DE) between 17 and 23 and a water activity (AW) below 0.1 comprising the steps of drying.

18. The method according to claim 17, wherein the drying is performed in a fluidized bed.

**Figure 1:** Percent OD per maximum OD as a function of time stirred in water.

**Figure 2:** % recovery as a function of Aw. pH=7.2. Lactobacillus acidophilus (NCFM) stability.

**Figure 3:** % recovery as a function of Aw, pH=7.2. Bifidobacterium lactis (Strain BBL) stability.

**Figure 4:** % recovery as a function of Aw. pH=7.2. Bifidobacterium Lactis (Strain BBI) stability

**Figure 5**. % recovery as a function of pH for Bifidobacterium lactis (Strain BBI), Bifidobacterium lactis (Strain BBL) and Lactobacillus acidophilus (NCFM strain).

Europäisches
Patentamt

European
Patent Office

Office européen
des brevets

**EUROPEAN SEARCH REPORT**

Application Number

EP 16 18 5880

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DAVID SEMYONOV ET AL: "Using ultrasonic vacuum spray dryer to produce highly viable dry probiotics", LWT- FOOD SCIENCE AND TECHNOLOGY, vol. 44, no. 9, 1 November 2011 (2011-11-01), pages 1844-1852, XP055196587, United Kingdom ISSN: 0023-6438, DOI: 10.1016/j.lwt.2011.03.021 * abstract * * paragraph [2.1.1] - paragraph [2.2.2] * * table 1 * | 1,2,5,6, 11,12, 15,17 | INV. A61K47/36 A61K9/14 A61K35/745 A61K35/747 A23L29/30 A23L33/135 C08L3/02 C08B30/18 |
| X | L. C. LI ET AL: "The effect of moisture content on the compression properties of maltodextrins", JOURNAL OF PHARMACY AND PHARMACOLOGY, vol. 42, no. 4, 12 April 1990 (1990-04-12), pages 272-275, XP055344455, LONDON; GB ISSN: 0022-3573, DOI: 10.1111/j.2042-7158.1990.tb05406.x * abstract * * page 272, left-hand column, paragraph 1 * * page 272, left-hand column, paragraph 2 - right-hand column, paragraph 3 * * figures 2,3,4 * | 1,17 | |
| X | CN 102 511 714 B (ZHEJIANG CONBA HEALTH PRODUCT CO LTD) 1 October 2014 (2014-10-01) * the whole document * | 1-18 | |

TECHNICAL FIELDS SEARCHED (IPC)

A61K
A23L
C08B
C08L

-/--

The present search report has been drawn up for all claims

| Place of search | Date of completion of the search | Examiner |
|---|---|---|
| The Hague | 13 February 2017 | Epskamp, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 16 18 5880

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | S Freers: "Maltodextrin"<br>In: "Handbook of Pharmaceutical Excipients - Fifth Edition",<br>2006, Pharmaceutical Press, UK / USA,<br>XP055075444,<br>ISBN: 978-0-85-369618-6<br>pages 442-444,<br>* paragraphs [0008], [0010] * | 1-18 | |

TECHNICAL FIELDS
SEARCHED (IPC)

The present search report has been drawn up for all claims

| Place of search | Date of completion of the search | Examiner |
|---|---|---|
| The Hague | 13 February 2017 | Epskamp, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 16 18 5880

This annex lists the patent family members relating to the patent documents cited in the above-mentioned European search report.
The members are as contained in the European Patent Office EDP file on
The European Patent Office is in no way liable for these particulars which are merely given for the purpose of information.

13-02-2017

| Patent document cited in search report | Publication date | Patent family member(s) | Publication date |
|---|---|---|---|
| CN 102511714 B | 01-10-2014 | NONE | |

For more details about this annex : see Official Journal of the European Patent Office, No. 12/82

EPO FORM P0459